# EUROPÄISCHE PATENTSCHRIFT

(11) **EP 0 870 183 B1**
(45) Veröffentlichungstag und Bekanntmachung des Hinweises auf die Patenterteilung: **16.02.2000**
(21) Anmeldenummer: 96944659.0
(22) Anmeldetag: 27.12.1996
(51) Int. Cl.: G01N 3/42, G01N 33/34

(54) **VORRICHTUNG UND VERFAHREN ZUM MESSEN DER OBERFLÄCHENHÄRTE VON GEWICKELTEN ROLLEN AUS PAPIER- ODER ANDEREN MATERIALBAHNEN**
DEVICE AND METHOD FOR MEASURING THE SURFACE HARDNESS OF ROLLS OF PAPER OR OTHER MATERIAL STRIPS
PROCEDE ET DISPOSITIF POUR MESURER LA DURETE DE LA SURFACE DE ROULEAUX DE BANDES DE PAPIER OU D'AUTRES MATERIAUX

(30) Priorität: 29.12.1995 DE 29520709 U
(43) Veröffentlichungstag der Anmeldung: 14.10.1998
(73) Patentinhaber: BELOIT TECHNOLOGIES, INC., Wilmington, Delaware 19808 (US)
(72) Erfinder: DÖRFEL, Walter, D-73087 Boll (DE); BOEHM, Gaston, D-73235 Weilheim (DE)
(74) Vertreter: Schumacher, Horst, Dr. Dipl.-Phys.
(86) Internationale Anmeldenummer: EP9605842
(87) Internationale Veröffentlichungsnummer: WO9724595

(56) Entgegenhaltungen:
- DE-A- 2 054 505
- DE-A- 4 105 115
- US-A- 4 586 517
- US-A- 5 282 382
- PATENT ABSTRACTS OF JAPAN vol. 010, no. 264 (P-495), 9.September 1986 & JP 61 090038 A (SANMITSUKU TSUSHO KK;OTHERS: 01), 8.Mai 1986,

## Beschreibung

Die Erfindung betrifft eine Vorrichtung und ein Verfahren zum Messen der Oberflächenhärte von gewickelten Rollen aus Papier- oder anderen Materialbahnen mit mindestens einem Härtemeßkopf mit mindestens einer drehbar gelagerten Kontaktrolle zur unter radialem Druck erfolgenden Anlage an der sich drehenden Rolle aus Papier oder dergleichen, bei der der Härtemeßkopf an einer Traverse im wesentlichen parallel zur Wickelachse der Rolle oder dergleichen verfahrbar ist und mit Mitteln zum Auswerten der Meßsignale des Härtemeßkopfes ausgestattet ist.

Beim Herstellen von Papier, Karton, Folien oder sonstigem bahnförmigem Material ergeben sich in Längs- und Querrichtung Unterschiede unter anderem in Dicke, Dichte und Flächengewicht (Profile). Herstellungsbedingt bleiben die Querprofile tendenziell meist gleich, d. h., die dicken oder dünnen Stellen liegen in Querrichtung meist an derselben Stelle.

Beim Aufwickeln dieser so hergestellten Bahnen zu Rollen bilden sich an den dicken Stellen gegenüber den dünneren Stellen Durchmesserunterschiede aus. Je größer eine Rolle aufgewickelt wird, desto größer wird in der Regel dieser Durchmesserunterschied.

Das Wickeln einer Rolle erfolgt üblicherweise im Kontakt mit einer oder mehreren Stütz- oder Tragwalzen. Dadurch ergibt sich an den dickeren Stellen eine höhere Kontaktkraft (Liniendruck, Nipkraft, Nipload) als an den Stellen mit geringerer Dicke.

Das heißt, die Lagen werden dem Profil entsprechend stark oder weniger stark aufeinandergepreßt. Diese Pressung ist als Härte an der Mantelfläche der Rolle meßbar.

Es ist gebräuchlich, diese Härte im Stillstand, d. h. bei fertig gewickelter Rolle zu messen. Dies geschieht üblicherweise, indem man z. B. manuell die Rückprallhärte in bestimmten Abständen quer zur Laufrichtung an der Mantelfläche mißt (z. B. Schmitthammermessung).

Aus der DE 20 54 505 B2 ist es bekannt, diese Messungen während des Auf- oder Abwickelns zu ermöglichen, und somit ein Härtequerprofil kontinuierlich oder in bestimmten, freiwählbaren Zeit-, Durchmesser- oder Lauflängenabständen aufzuzeigen. Hierzu wird eine einzige Kontaktrolle mit zwei unterschiedlich großen piezoelektrischen Meßumformern, die in die Rollenoberfläche eingearbeitet sind, verwendet.

Um Parameter einer zur Rolle gewickelten Materialbahn, wie Rollenhärte, Bahnspannung, Uniformität des Rollendurchmessers entlang der Rollenbreite und ähnliche, während des Wickelvorganges genauer überwachen und gegebenenfalls beeinflussen zu können, wird vorgeschlagen, erste und zweite Mittel vorzusehen, mit denen mindestens eine der Kontaktrollen mit einer ersten Kraft in Anlage an der Rolle haltbar ist und mit denen mindestens eine der Kontaktrollen mit einer zweiten Kraft tiefer als mit der ersten Kraft in die Oberfläche eindringbar ist. Mit einer erfindungsgemäßen Vorrichtung kann die Oberflächenhärte von gewickelten Rollen aus Papier oder dergleichen dadurch gemessen werden, daß die Differenz der Eindringtiefe oder des Rückpralls mindestens einer an der Wickeloberfläche der sich drehenden Rolle aus Papier oder dergleichen mit etwa radialer Druckkomponente in Anlage gehaltenen Kontaktrolle entlang der Wickelachse der Rolle fortlaufend oder intermittierend gemessen wird.

Hierdurch wird eine Messung des Oberflächenhärteprofils quer zur Bahnrichtung während des Wickelprozesses auf einfache Weise möglich. Dabei werden eine Vielzahl von Daten gewonnen, die für die Optimierung des Herstellungs- oder Verarbeitungsprozesses zur Qualitäts- oder Quantitätssteigerung des Endproduktes genutzt werden können:

Eine Nutzungsmöglichkeit der Meßdaten besteht z. B. darin, die Längsschneidevorrichtungen an einer Rollenschneidemaschine (Slitter) in Abhängigkeit des gemessenen Härteprofils so einzustellen, daß die gewickelten Teilbahnen in sich jeweils ein homogeneres Härteprofil erhalten und/oder das Risiko von Bahnrissen vermindert wird. Die Wickelgeschwindigkeit kann somit erhöht werden.

Die Meßköpfe können nun in verschiedener Weise ausgestaltet sein:

Bei einer ersten und zweiten Ausführungsform besteht der Härtemeßkopf aus mindestens zwei mit seitlichem Abstand, vorzugsweise in ihrer Achsrichtung, nebeneinander angeordneten, mit unterschiedlicher Kraft gegen die zu messende Rolle andrückbaren Kontaktrollen und Mitteln zur Messung der Eindringtiefendifferenz der Kontaktrollen. Gemäß einer dritten Ausführungsform ist der Härtemeßkopf mit mindestens einem Stoßerzeuger zum radialen Vortreiben der Kontaktrolle gegen die Wickeloberfläche und zum Messen des Rückpralls der Kontaktrolle versehen. Einzelheiten der genannten Ausführungsformen ergeben sich aus den im Zusammenhang mit der Zeichnung erläuterten Ausführungsbeispielen.

Die vorgenannten sowie die beanspruchten und in den Ausführungsbeispielen beschriebenen, erfindungsgemäß zu verwendenden Verfahrensschritte sowie Bauteile unterliegen hinsichtlich ihrer Verfahrensbedingungen, ihrer Größe, Formgestaltung, Materialauswahl und technischen Konzeption keinen besonderen Ausnahmebedingungen, so daß die in dem jeweiligen Anwendungsgebiet bekannten Auswahlkriterien uneingeschränkt Anwendung finden können.

Weitere Einzelheiten, Merkmale und Vorteile des Gegenstandes der Erfindung ergeben sich aus der nachfolgenden Beschreibung der zugehörigen Zeichnung, in der - beispielhaft - bevorzugte Ausführungsformen dargestellt sind. In der Zeichnung zeigen
- Fig. 1: eine Rollenwickelmaschine (schematisch) in Stirnseitenansicht mit Härtemeßeinrichtung (Ansicht A gemäß Fig. 2);
- Fig. 2: dieselbe Vorrichtung in Frontansicht (Ansicht B gemäß Fig. 1);
- Fig. 3A/B: für dieselbe Vorrichtung ein erster Härtemeßkopf in Frontalansicht (Fig. 3A - entsprechend der Blickrichtung C gemäß Fig. 3B) und - Fig. 3B - in Stirnseitenansicht (entsprechend Ansicht D in Fig. 3A);
- Fig. 4A/B: eine zweite Ausführungsform eines Härtemeßkopfes in derselben Darstellungsart wie in Fig. 3A/B sowie
- Fig. 5: eine dritte Ausführungsform eines Härtemeßkopfes in der Darstellungsart wie Fign. 3B und 4B.

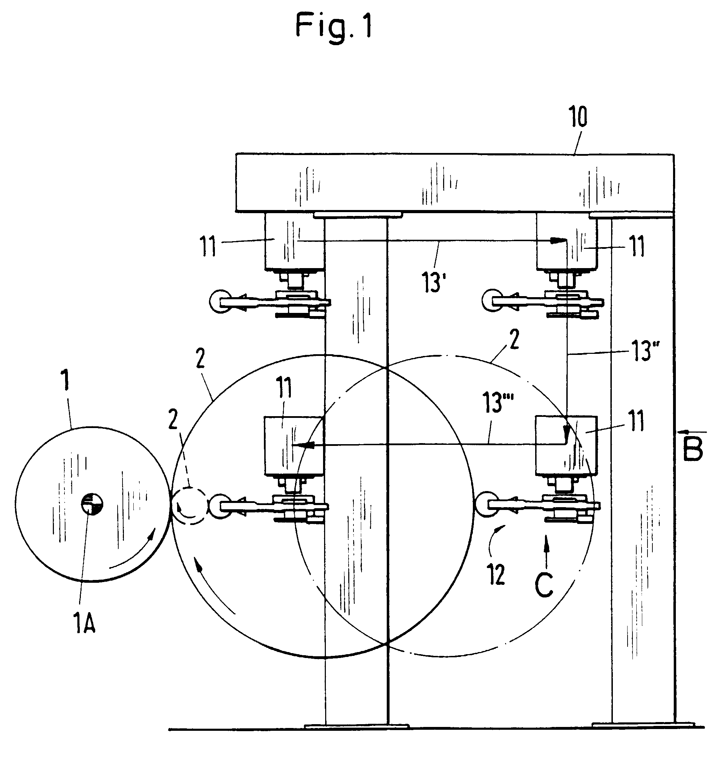

In der in Fig. 1 dargestellten Wickelmaschine ist eine angetriebene Stützwalze 1 vorgesehen, an der eine Rolle 2 aus einer Papierbahn oder dergleichen mit hier nicht einzeln dargestellten, da ansich bekannten Tragarmen in Anlage gehalten wird. Wie aus Fig. 1 in gestrichelter Darstellung ersichtlich, ist der Durchmesser der zu wickelnden Rolle 2 anfänglich sehr gering. Er nimmt nachfolgend auf ein bestimmtes Endmaß (mit durchgezogener Linie dargestellt) zu, um danach aus der Wickelposition entfernt zu werden (siehe strichpunktierte Darstellung der Rolle 2).

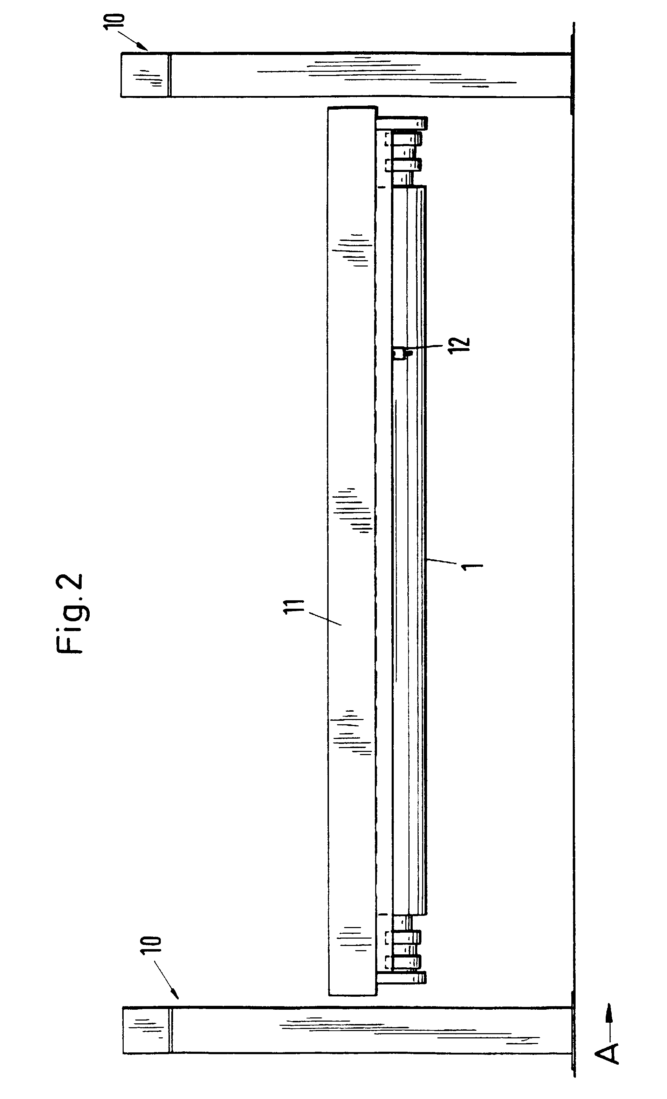

Ein aus Pfosten und Querträgern bestehendes Gestell 10 trägt eine Traverse 11, die sich über die gesamte Maschinenbreite erstreckt (wie aus Fig. 2 ersichtlich). Die Traverse 11 ist an dem paarweise ausgebildeten Gestell 10 derart befestigt und geführt gelagert, daß die etwa parallel zur Wickelachse 1A angeordnete Traverse 10 in mehrere zur Wickelachse 1A rechtwinklig orientierte Richtungen mit horizontaler und vertikaler Richtungskomponente verfahrbar ist. Dies ist in Fig. 1 durch die viermalige Darstellung der Traverse 11 und des noch zu erläuternden Härtemeßkopfes 12 in Verbindung mit den Bewegungslinien 13', 13'', 13''' der Traverse 11 kenntlich gemacht. Auch wenn die Traverse 11 und der Härtemeßkopf 12 in den vier Positionen (lediglich der Übersichtlichkeit halber) mit ausgezogenen Linien dargestellt ist, so befindet sich diese Baueinheit doch jeweils nur in einer der bezüglich des Gestelles 10 realisierbaren Positionen.

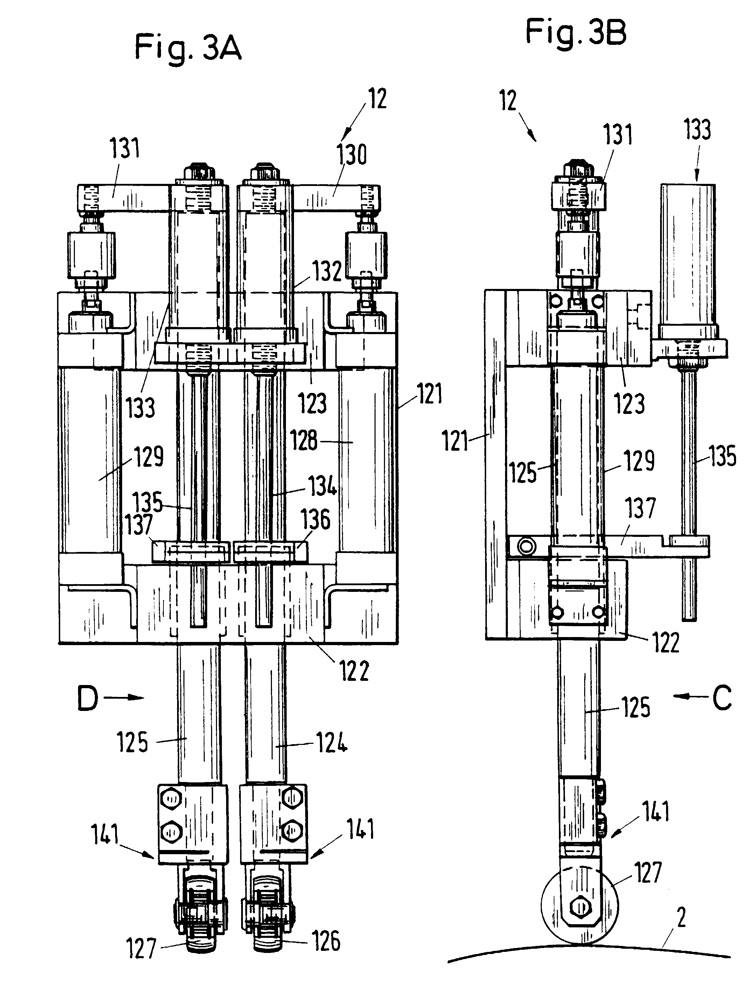

An der Traverse 11 ist der Härtemeßkopf 12 derart geführt, daß der Härtemeßkopf entlang der gesamten Maschinenbreite, d.h. entlang der gesamten Wickelbreite verfahrbar ist, so daß ein Härteprofil quer zur Bahnlaufrichtung an der Rollenoberfläche erstellt werden kann. Der Härtemeßkopf 12 arbeitet bevorzugt nach einer der drei in den Fign. 3A bis 5 wiedergegebenen Arbeitsweisen:

Gemäß Fign. 3A, 3B weist ein Härtemeßkopf 12 eine Grundplatte 121 auf, die in im einzelnen nicht dargestellter Weise mit Führungsmitteln zum Verfahren entlang der Traverse 11 an ihrer Rückseite (in Fig. 3B also links) versehen ist. Die Grundplatte trägt zwei voneinander beabstandete Führungskörper 122 und 123, die in Führungsbohrungen Meßfühlerstempel 124 und 125 gleitend führen. An dem meßpunktseitigen Ende trägt jeder Meßfühlerstempel eine kugelgelagerte Kontaktrolle 126, 127, die an der Oberfläche des hinsichtlich seines Härteprofils zu messenden Rolle 2 in achsparalleler Ausrichtung und in Achsrichtung voneinender beabstandet in Anlage gehalten wird. Hierzu dient, für jeden Meßfühlerstempel einzeln, ein Linearantrieb 128, 129, der jeweils über ein Joch 130, 131 mit dem den Kontaktrollen 126, 127 gegenüberliegenden Stirnende der Meßfühlerstempel 124, 125 druck- und zugfest, d.h. im Antriebssinne verbunden sind. Außerdem sind an der Außenseite des Führungskörpers 123 zwei Wegmeßgeräte 132, 133 befestigt, deren axialbewegliche Wegaufnehmerstempel 134, 135 starr über Klemmen 136, 137 mit den Meßfühlerstempeln 124 respektive 125 verbunden sind. Die Arbeitweise dieses Härtemeßkopfes ist derart, daß beide Kontaktrollen 126, 127 mit unterschiedlichem Druck gegen die Oberfläche der zu messenden Rolle 2 bezüglich der Traverse 11 vorgeschoben werden.

Eine der beiden Kontaktrollen 126 bzw. 127 dient zur Referenzmessung. Die Kontaktkraft zwischen dieser Kontaktrolle und der zu messenden Rolle ist klein und soll nur gerade so groß sein, um die Papierlagen satt, d. h. ohne Lufteinschlüsse, aufeinander zu legen, ohne diese elastisch oder plastisch zu verdichten. Die so erreichte Position wird mittels des zugeordneten Wegaufnehmers 132 bzw. 133 gemessen.

Die andere der beiden Kontaktrollen 126 bzw. 127 wird für die eigentliche Messung der Eindringtiefe mit höherer Kontaktkraft beaufschlagt. Die Kontaktkraft dieser Rolle solle jedoch, um Beschädigungen des Papiers zu vermeiden, höchstens dessen Elastizität ausschöpfen.

Aus den gemessenen Wegen wird durch eine hier nicht gezeigte elektronische Einrichtung ein Differenzwert gebildet, der als Funktion der Härte definiert und skaliert wird.

Durch die Referenzmessung werden außerdem Fehler, wie z. B. Unparallelität zwischen Rolle 2 und Traverse 11 oder Durchbiegung von der Traverse 11 oder Konizität der Rolle 2, eliminiert.

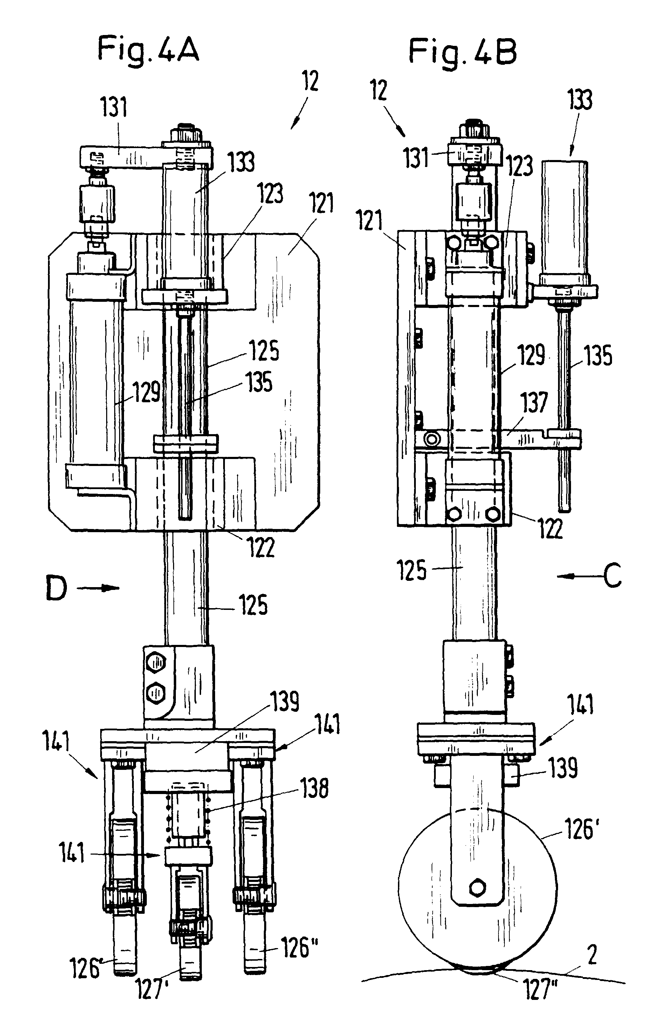

Bei dem Ausführungsbeispiel nach Fig. 4A und 4B ist an der Grundplatte 121 nur ein einziger Meßfühlerstempel 125 in den Führungskörpern 122 und 123 widerstandsarm gleitend gelagert. Mit dem einzigen Linearantrieb 129 wird der Meßfühlerstempel 125 über das Joch 131 axial an die zu messende Rolle 2 aus Papier oder dergleichen herangefahren und eine bestimmte Andrückkraft ausgeübt. Bei diesem Ausführungsbeispiel sind drei Kontaktrollen 126', 126'' und 127' in zur Rolle 2 etwa achsparalleler Anordnung drehbar wälzgelagert. Die mittlere der drei seitlich beabstandeten Kontaktrollen ist bezüglich des Meßfühlerstempels 125 gegen den Druck einer Schraubendruckfeder 138 verlagerbar. Im entspannten Zustand ragt die Lauffläche der Kontaktrolle 127' im Vergleich zu der Lauffläche der Kontaktrollen 126' und 126'' vor. Wird nun der Meßfühlerstempel 125 mit Axialdruck gegen die Oberfläche der Rolle 2 (in der Regel rechtwinklig zu derselben) vorgeschoben, bis die Kontaktrollen 126', 126'' mit Andruck an der Rollenoberfläche anliegen, wird die Feder 138 je nach Oberflächen-Härteunterschied im Bereich der drei Kontaktrollen mehr oder minder weit in Richtung auf das Joch 131 zusammengedrückt. Eine Kraftmeßzelle 139 erfaßt die Federkraft der Feder 138, die sich entsprechend des Oberflächenhärteprofils an der Meßstelle einstellt.

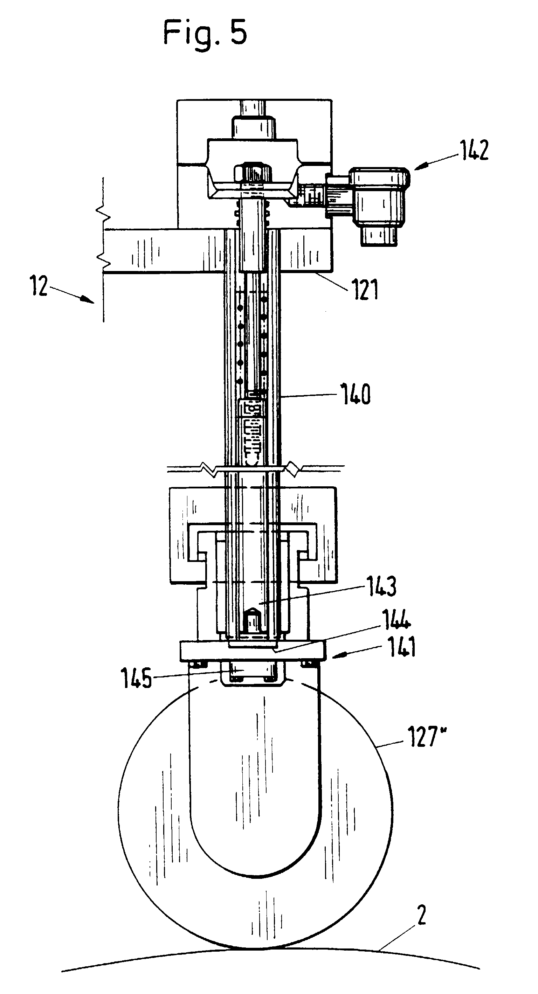

Bei der dritten Ausführungsform (nach Fig. 5) trägt eine Grundplatte 121 über ein damit starr verbundenes Rohr 140 einen gabelförmigen Halter 141 für eine kugelgelagert drehbare Kontaktrolle 127''. Ein Schwingungsgenerator 142 treibt innerhalb des Rohres 140 einen Stößel oder Hammer 143 gegen einen Amboß 144. Dadurch wird die Kontaktrolle 127'' gegen die Oberfläche der Rolle 2 kurzzeitig vorgetrieben. Je nach Oberflächenhärte prallt die Kontaktrolle 127'' stärker oder weniger stark zurück. Die Stärke dieses Rückpralls kann z. B. durch eine Beschleunigungsmeßzelle 145 als Funktion der Oberflächenhärte der Rolle 2 gemessen werden.

Im übrigen können, wie der Fig. 1 entnehmbar, größeren Rollendurchmesseränderungen, die den Hubweg der Härtemeßköpfe überschreiten, durch radiales Bewegen der gesamten Traverse samt Härtemeßkopf Rechnung getragen werden. Weitere Bewegungsmöglichkeiten der Traverse dienen dazu, die fertige Rolle aus der Wickelmaschine heraustransportieren zu können.

Bei allen Ausführungsformen kann mit Hilfe einer Positionsmeßeinrichtung zum Erfassen der Position des Härtemeßkopfes entlang der Traverse bzw. der zu messenden Rolle auch eine etwaige Dickenänderung der Rolle in Bahnquerrichtung, wie z. B. eine Konizität der Rolle, erfaßt und ausgewertet werden.

### Bezugszeichenliste

- 1: Stützwalze
- 1A: Wickelachse
- 2: Papierrolle
- 10: Gestell
- 11: Traverse
- 12: Härtemeßkopf
- 13': Bewegungslinie
- 13'': Bewegungslinie
- 13''': Bewegungslinie
- 121: Grundplatte
- 122: Führungskörper
- 123: Führungskörper
- 124: Meßfühlerstempel
- 125: Meßfühlerstempel
- 126: Kontaktrolle
- 126': Kontaktrolle
- 126'': Kontaktrolle
- 127: Kontaktrolle
- 127': Kontaktrolle
- 127'': Kontaktrolle
- 128: Linearantrieb
- 129: Linearantrieb
- 130: Joch
- 131: Joch
- 132: Wegmeßgerät
- 133: Wegmeßgerät
- 134: Wegaufnahmestempel
- 135: Wegaufnahmestempel
- 136: Klemme
- 137: Klemme
- 138: Schraubendruckfeder
- 139: Krafmeßzelle
- 140: Rohr
- 141: Halter
- 142: Schwingungsgenerator
- 143: Hammer
- 144: Amboß
- 145: Beschleunigungsmeßzelle

## Patentansprüche

1. Vorrichtung zum Messen der Oberflächenhärte von gewikkelten Rollen (2) aus Papier- oder anderen Materialbahnen, mit mindestens einem Härtemeßkopf (12) mit mindestens einer drehbar gelagerten Kontaktrolle zur unter radialem Druck erfolgenden Anlage an der sich drehenden Rolle (2) aus Papier oder dergleichen, bei der der Härtemeßkopf (12) an einer Traverse (11) im wesentlichen parallel zur Wickelachse der Rolle (2) oder dergleichen verfahrbar ist und mit Mitteln zum Auswerten der Meßsignale des Härtemeßkopfes (12) ausgestattet ist,
**dadurch gekennzeichnet, daß**
erste und zweite Mittel vorgesehen sind, mit denen mindestens eine der Kontaktrollen (126, 127; 126', 126'', 127'') mit einer ersten Kraft in Anlage an der Rolle (2) haltbar ist und mit denen mindestens eine der Kontaktrollen (126, 127; 127', 127'') mit einer zweiten Kraft tiefer als mit der ersten Kraft in die Oberfäche eindringbar ist.

2. Vorrichtung nach Anspruch 1, **dadurch gekennzeichnet**, daß die Traverse (11) etwa rechtwinklig zur Wickelachse, insbesondere entsprechend der Änderung des Wickeldurchmessers der Rolle (2) aus Papier oder dergleichen, mit dem mindestens einen Härtemeßkopf (12) als Ganzes verfahrbar an einem Gestell (10) befestigt ist.

3. Vorrichtung nach Anspruch 1 oder 2, **dadurch gekennzeichnet, daß** der Härtemeßkopf (12) mindestens zwei mit seitlichem Abstand voneinander angeordneten Kontaktrollen (126, 127, 126', 127', 126'') und Mitteln (132, 133) zur Messung der Eindringtiefendifferenz der Kontaktrollen versehen ist.

4. Vorrichtung nach Anspruch 1 oder 2, **dadurch gekennzeichnet**, daß der Härtemeßkopf (12) mit mindestens einem Stoßerzeuger (142, 143, 144) zum radialen Vortreiben der Kontaktrolle (127'') gegen die Wickeloberfläche und zum Messen des Rückpralls (145) versehen ist.

5. Vorrichtung nach einem der Ansprüche 1 bis 4, **gekennzeichnet durch** Mittel zur Positionserfassung des Härtemeßkopfes entlang der Rollenbreite.

6. Verfahren zum Messen der Oberflächenhärte von gewickelten Rollen aus Papier oder dergleichen, mit einer Vorrichtung nach einem der Ansprüche 1 bis 5,
**dadurch gekennzeichnet, daß**
die Differenz der Eindringtiefe oder des Rückpralls mindestens einer an der Wickeloberfläche der sich drehenden Rolle (2) aus Papier oder dergleichen mit etwa radialer Druckkomponente in Anlage gehaltenen Kontaktrolle bei zwei verschiedenen etwa radialen Anlagekräften entlang der Wickelachse der Rolle fortlaufend oder intermittierend gemessen wird.

7. Verfahren nach Anspruch 6, **dadurch gekennzeichnet**, daß das von dem Härtemeßkopf erfaßte Oberflächenhärteprofil an die Herstellungsmaschine, an eine Längsteilmaschine oder dergleichen zur Qualitäts- und/oder Quantitätsverbesserung weitergegeben wird.

## Claims

1. A device for measuring the surface hardness of wound rolls (2) of paper or other material webs, comprising at least one hardness measuring head (12) with at least one rotatably mounted contact wheel for resting against the rotating roll (2) of paper or the like under radial pressure, wherein the hardness measuring head (12) is displaceable on a traverse (11) substantially parallel to the winding axis of the roll (2) or the like, and is provided with means for evaluating the measuring signals from the hardness measuring head (12), characterised in that first and second means are provided, with which at least one of the contact wheels (126, 127; 126', 126", 127") can be held against the roll (2) by a first force, and with which at least one of the contact wheels (126, 127; 127', 127") can penetrate the surface with a second force more deeply than with the first force.

2. A device according to claim 1, characterised in that the traverse (11) with the at least one hardness measuring head (12) is fixed as a whole to a frame (10) so as to be displaceable substantially at right angles to the winding axis, in particular in accordance with the change in the winding diameter of the roll (2) of paper or the like.

3. A device according to claim 1 or 2, characterised in that the hardness measuring head (12) is provided with at least two laterally spaced contact wheels (126, 127, 126', 127', 126") and with means (132, 133) for measuring the difference in the penetration depth of the contact wheels.

4. A device according to claim 1 or 2, characterised in that the hardness measuring head (12) is provided with at least one impact generator (142, 143, 144) for driving the contact wheel (127") radially towards the winding surface and for measuring the rebound (145).

5. A device according to any one of claims 1 to 4, characterised by means for detecting the position of the hardness measuring head along the width of the roll.

6. A method of measuring the surface hardness of wound rolls of paper or the like using a device according to any one of claims 1 to 5, characterised in that, for two different, substantially radial contact forces, the difference in the penetration depth or rebound of at least one contact wheel (1) held resting against the winding surface of the rotating roll (2) of paper or the like with a substantially radial pressure component is measured along the winding axis of the roll continuously or intermittently.

7. A method according to claim 6, characterised in that the surface hardness profile detected by the hardness measuring head is transmitted to the production machine, a longitudinal cutting machine or the like, in order to improve quality and/or quantity.

## Revendications

1. Dispositif pour mesurer la dureté de la surface de rouleaux (2) enroulés, constitués de bandes de papier ou d'un autre matériau, avec au moins une tête de mesure de dureté (12), équipée d'au moins un rouleau de contact monté à rotation, pour assurer un appui, accompagné de pression radiale, sur le rouleau (2) de papier ou analogue, à rotation, dans lequel la tête de mesure de dureté (12) est déplaçable sur une traverse (11), sensiblement parallèlement à l'axe d'enroulement du rouleau (2) ou analogue, et est équipée de moyens pour évaluer les signaux de mesure issus de la tête de mesure de dureté (12),
caractérisé en ce que
sont prévus des premier et deuxième moyens, à l'aide desquels au moins l'un des rouleaux de contact (126, 127 ; 126', 126", 127") est susceptible d'être fixé avec une première force, en appui sur le rouleau (2), et à l'aide desquels au moins l'un des rouleaux de contact (126, 127 ; 127', 127") peut s'enfoncer dans la surface, avec une deuxième force, à une profondeur supérieure à celle provoquée par la première force.

2. Dispositif selon la revendication 1, caractérisé en ce que la traverse (11) est fixée d'un tout avec la au moins une tête de mesure de dureté (12). de façon déplaçable sur un bâti (10), à peu près perpendiculairement par rapport à l'axe d'enroulement, en particulier de manière correspondante à la variation de diamètre d'enroulement du rouleau (2) en papier ou analogue.

3. Dispositif selon la revendication 1 ou 2, caractérisé en ce que la tête de mesure de dureté (12) est dotée d'au moins deux rouleaux de contact (126, 127 ; 126', 127', 126"), disposés à distance latérale mutuelle, et de moyens (132, 133) pour mesurer la différence de profondeur de pénétration des rouleaux de contact.

4. Dispositif selon la revendication 1 ou 2, caractérisé en ce que la tête de mesure de dureté (12) est dotée d'au moins un générateur de choc (142, 143, 144) pour avancer radialement le rouleau de contact (127") contre la surface d'enroulement et pour mesurer le rebond (145).

5. Dispositif selon l'une des revendications 1 à 4, caractérisé par des moyens destinés à appréhender la position de la tête de mesure de dureté sur la largeur du rouleau.

6. Procédé de mesure de la surface de rouleaux en papier ou analogue, enroulés, à l'aide d'un dispositif selon l'une des revendications 1 à 5,
caractérisé en ce que
la différence des profondeurs de pénétration ou du rebond d'au moins un rouleau de contact, lorsque l'on a deux efforts d'application différents, orientés à peu près radialement, rouleau de contact maintenu en appui avec une composante de pression orientée à peu près radialement sur la surface d'enroulement du rouleau (2) en rotation et constitué en papier ou analogue, est mesurée de façon continue ou intermittente, en progressant le long de l'axe d'enroulement du rouleau.

7. Procédé selon la revendication 6, caractérisé en ce que le profil de dureté de surface appréhendé par la tête de mesure de dureté est retransmis à la machine de fabrication, à une machine de subdivision longitudinale ou analogue, dans le but d'obtenir une amélioration qualitative et/ou quantitative.
